# DEMANDE DE BREVET EUROPEEN

(11) **EP 1 247 517 A2**
(43) Date de publication de la demande: **09.10.2002**
(21) Numéro de dépôt: 02290768.7
(22) Date de dépôt: 27.03.2002
(51) Int. Cl.: A61K 7/043

(54) **Composition cosmétique réticulant à l'humidité**

(30) Priorité: 06.04.2001 FR 0104680
(71) Demandeur: L'OREAL, 75008 Paris (FR)
(72) Inventeur: Bernard, Pascale, 94370 Sucy-en-Brie (FR); Ramin, Roland, 75014 Paris (FR); Mondet, Jean, Aulnay-Sous-Bois (FR)
(74) Mandataire: Casalonga, Axel

(57) **Abrégé**

L'invention concerne l'utilisation comme vernis à ongle d'une composition cosmétique réticulant à l'humidité comprenant, dans un milieu anhydre cosmétiquement acceptable, et exempt de composés à atomes d'hydrogène labiles, i) soit
a) au moins un premier composé, appelé composé A, comportant au moins deux fonctions réactives non bloquées X, et
b) au moins un deuxième composé, appelé composé B, comportant au moins deux fonctions réactives bloquées Y, susceptibles d'être débloquées sous l'action de l'humidité de manière à pouvoir réagir avec les fonctions réactives non bloquées X du ou des composés A,
   ii) soit au moins un composé comportant à la fois au moins deux fonctions réactives non bloquées X et au moins deux fonctions réactives non bloquées Y, susceptibles d'être débloquées sous l'action de l'humidité de manière à pouvoir réagir avec les fonctions réactives non bloquées X,
la fonctionnalité moyenne du système, c'est-à-dire le nombre total de fonctions réactives non bloquées X et de fonctions réactives bloquées Y rapporté au nombre total de molécules de composés étant strictement supérieure à 2.

## Description

La présente invention concerne des compositions cosmétiques réticulant à l'humidité, en particulier des vernis à ongles, ainsi qu'un procédé de revêtement des matières kératiniques à partir de ces compositions.

Il est connu du domaine des peintures que la réticulation d'un dépôt polymérique améliore considérablement sa résistance mécanique (résistance au frottement et aux chocs) et chimique (résistance aux solvants ou huiles).

Les dépôts sur les ongles sont obtenus généralement par simple séchage - n'impliquant aucune réaction chimique - de compositions cosmétiques contenant principalement des pigments ou colorants, des polymères filmogènes et des solvants organiques volatiles.

Ces dépôts ne présentent pas toujours une tenue satisfaisante et présentent l'inconvénient de nécessiter un renouvellement à des intervalles rapprochés ce qui représente une contrainte désagréable pour l'utilisatrice.

Il est connu, par la demande NL-A-6911125, des revêtements pigmentés pour le domaine dentaire ou les vernis à ongles contenant des prépolymères d'uréthanes portant des groupes NCO libres qui durcissent à l'application avec l'humidité de l'air. Mais la réticulation de ces compositions est directement liée à la quantité d'humidité, si bien qu'il est impossible de contrôler le taux de réticulation du film obtenu.

La demanderesse a découvert qu'il était possible d'obtenir des vernis présentant de bonnes propriétés de résistance chimique et mécanique en procédant à la réticulation, en particulier de prépolymères d'uréthane, avec des composés multifonctionnels comportant des groupes réactifs. Ces compositions cosmétiques, et en particulier les vernis à ongles, sont susceptibles d'être réticulées *in situ* et de former ainsi des films présentant les propriétés précitées.

Dans cette perspective, il apparaît difficile du point de vue pratique d'envisager l'utilisation de deux réactifs conservés dans des récipients séparés et mélangés immédiatement avant l'application.

Le stockage, dans un même récipient, de deux réactifs susceptibles de réagir l'un avec l'autre à un moment précis pose alors le problème de la réticulation prématurée du système qu'il s'agit à tout prix d'éviter.

La demanderesse a résolu ce problème en bloquant un des deux types de fonctions réactionnelles impliquées dans la réaction de réticulation. Les fonctions bloquées sont débloquées, après application de la composition cosmétique sur le substrat, sous l'action de l'humidité, et les fonctions ainsi débloquées peuvent alors réagir avec les fonctions réactives non bloquées des composés également présents dans la composition.

Ces compositions cosmétiques réticulant à l'humidité présentent une bonne stabilité dans le temps, et permettent l'obtention de films réticulés présentant une bonne résistance à l'eau, aux solvants tels que les parfums ou huiles, aux frottements et aux chocs, évitant ainsi toute usure ou écaillage.

La présente invention a par conséquent pour objet une composition cosmétique réticulant à l'humidité comprenant des composants à fonctions réactives, bloquées pour certaines et non bloquées pour d'autres, les fonctions bloquées étant susceptibles d'être débloquées à l'humidité.

L'invention a également pour objet un procédé de revêtement d'un substrat kératinique.

Un autre objet est constitué par l'utilisation comme vernis à ongle des compositions précitées.

D'autres objets de l'invention apparaîtront à la lecture de la description et des exemples qui suivent.

La composition objet de l'invention est une composition cosmétique réticulant à l'humidité comprenant, dans un milieu anhydre cosmétiquement acceptable, et exempt de composés à atomes d'hydrogène labiles,
i) soit
   a) au moins un premier composé, appelé composé A, comportant au moins deux fonctions réactives non bloquées X, et
   b) au moins un deuxième composé, appelé composé B, comportant au moins deux fonctions réactives bloquées Y, susceptibles d'être débloquées sous l'action de l'humidité de manière à pouvoir réagir avec les fonctions réactives non bloquées X du ou des composés A,
ii) soit au moins un composé comportant à la fois au moins deux fonctions réactives non bloquées X, et au moins deux fonctions réactives bloquées Y, susceptibles d'être débloquées sous l'action de l'humidité de manière à pouvoir réagir avec les fonctions réactives non bloquées X.

Pour que le système réactif formé par les composés A et B ou par le ou les composés portant les fonctions X et Y puisse former un réseau macromoléculaire réticulé, sa fonctionnalité moyenne, c'est-à-dire le nombre total de fonctions réactives non bloquées X et de fonctions réactives bloquées Y rapporté au nombre total de molécules de composés doit être strictement supérieure à 2. En effet, une fonctionnalité moyenne inférieure ou égale à 2 donnerait simplement un système polymérique linéaire ou ramifié.

Pour l'obtention d'un effet de réticulation satisfaisant, la fonctionnalité moyenne du système réticulant des compositions cosmétiques de la présente invention est de préférence au moins égale à 2,2, notamment allant de 2,2 à 100, et mieux encore allant de 2,5 à 100.

Dans un mode de réalisation des compositions cosmétiques de la présente invention, une fraction plus ou moins importante ou la totalité des composés A et/ou B peuvent porter respectivement, en plus des fonctions réactives non bloquées X et/ou des fonctions réactives non bloquées Y qui leur sont propres, une ou plusieurs fonctions "coréactives". Autrement dit, une partie ou la totalité des composés A peuvent porter, en plus des fonctions réactives non bloquées X, une ou plusieurs fonctions réactives bloquées Y et, de manière analogue, une partie ou la totalité des composés B peuvent porter, en plus des fonctions réactives bloquées Y, une ou plusieurs fonctions réactives non bloquées X.

Dans un mode de réalisation particulier de la présente invention, l'ensemble des composés formant le système de réticulation présent dans la composition comporte à la fois des fonctions réactives non bloquées X et des fonctions réactives bloquées Y.

Selon un mode de réalisation de l'invention, l'humidité assurant la réticulation peut provenir de l'air et/ou du support sur lequel est appliquée ladite composition.

Selon la présente invention, les fonctions réactives non bloquées X sont choisies notamment parmi les fonctions isocyanate et époxyde, et les doubles liaisons éthyléniques.

### Les composés à fonctions isocyanates :

Les composés comportant au moins deux fonctions isocyanate libres sont connus dans la technique. Il peut s'agir de polyisocyanates pouvant avoir une faible masse moléculaire inférieure à 1000000, y compris des diisocyanates, triisocyanates ou polyisocyanates pouvant avoir une masse moléculaire inférieure à 10000. Ces polyisocyanates sont généralement obtenus par polyaddition, polycondensation et/ou greffage, portant deux ou plus de deux fonctions isocyanates soit aux extrémités de chaîne ou sur les groupes latéraux. Les polyisocyanates peuvent être linéaires, ramifiés, aliphatiques, cycloaliphatiques ou aromatiques.

On peut citer à titre d'exemples de tels composés :
a) Les diisocyanates comportant de 4 à 50 atomes de carbone, de préférence de 4 à 30, tels que le 1,4-tétraméthylène diisocyanate, le 1,6-hexaméthylènediisocyanate, le 2,6- et le 2,4-toluènediisocyanate, le diphénylméthanediisocyanate, et l'isophoronediisocyanate.
b) les triisocyanates de formule 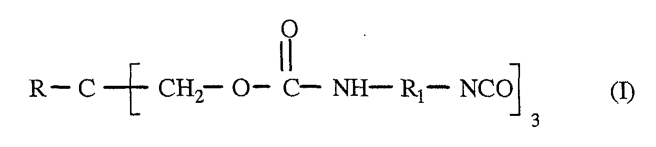 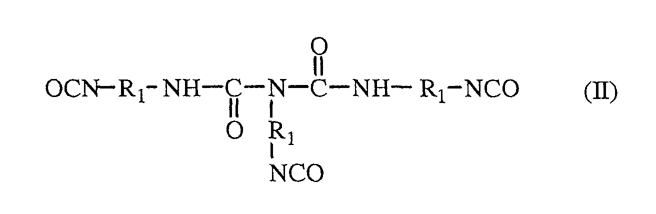 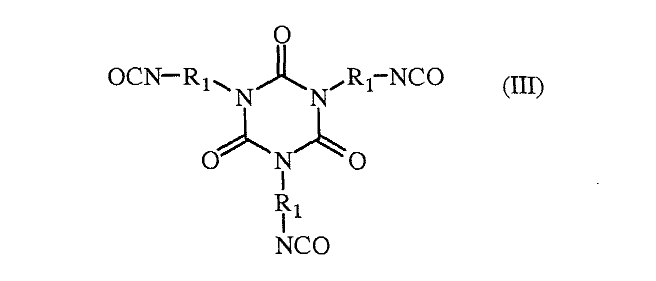 où R est un radical alkyle comportant de 1 à 30 atomes de carbone, chaque R₁ représente indépendamment un radical divalent hydrocarboné linéaire, ramifié ou cyclique ayant de 2 à 30 atomes de carbone.
c) les polycondensats à groupes isocyanate terminaux ou latéraux tels que les polyuréthannes et/ou polyurées (y compris les copolymères séquencés comportant au moins une séquence polyuréthanne et/ou polyurée et au moins une séquence polyéther, polyester, polysiloxane, alkyde ou polyacrylate), ainsi que les polyesters, polyamides, polyépoxy, polyéthers, perfluoropolyéthers.
d) les polymères résultant de la copolymérisation de monomères vinyliques, allyliques et/ou (méth)acryliques et de comonomères à insaturation éthylénique comportant une fonction isocyanate libre comme le méthacrylate de 2-isocyanato éthyle.

Comme polyisocyanate, on peut utiliser le DESMODUR® N de la société BAYER, le TOLONATE® HDB-LV de la société RHODIA.

### Les composés à fonction époxyde :

Les composés comportant au moins deux fonctions époxyde sont connus de l'état de la technique. Ils peuvent être de toute nature chimique. Il peut s'agir de diépoxydes ou de polyépoxydes de faibles masses (inférieures ou égales à 5000), ou bien d'oligomères ou de polymères de toute nature chimique, obtenus par polyaddition, polycondensation et/ou greffage, portant au moins deux fonctions époxydes libres, soit aux extrémités de chaînes, soit en groupes latéraux. On peut citer à titre d'exemples de tels composés :
a) le diglycidyl éther de bisphénol A résultant de la condensation entre le bisphénol A et l'épichlorhydrine, de structure 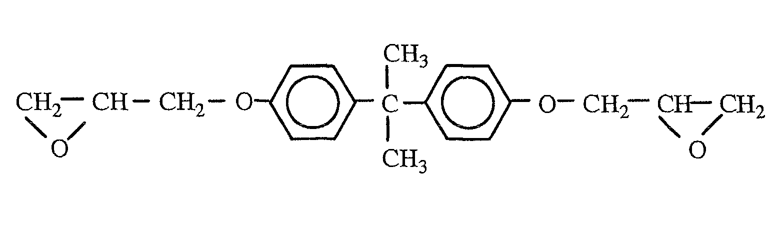
b) les résines diépoxy résultant de la condensation supérieure entre l'éther diglycidique de bisphénol A et l'épichlorhydrine,
c) les résines époxyester à extrémités α,ω-diépoxy résultant de la condensation d'un diacide carboxylique ayant notamment de 2 à 60 atomes de carbone avec un excès stoechiométrique des composés a) ou b),
d) les résines époxyéthers à extrémités α,ω-diépoxy résultant de la condensation d'un diol ayant notamment de 2 à 60 atomes de carbone avec un excès stoechiométrique des composés a) ou b),
e) les huiles naturelles ou synthétiques portant au moins deux groupes époxydes, comme par exemple l'huile de soja époxydée, l'huile de lin époxydée, l'huile de vernonia, notamment décrites dans la demande EP-A-645134,
f) les oligomères ou polymères résultant de la copolymérisation de monomères insaturés ou vinyliques, allyliques et/ou (méth)acryliques, et de comonomères à insaturation éthylénique comportant une fonction époxyde libre (comme le méthacrylate de glycidyle),
g) les autres polycondensats à groupes époxy terminaux et/ou latéraux tels que les polyesters, polyesteramides, polyamides, alkydes, polyuréthanes et/ou polyurées, polyéthers et perfluoroplyéthers ou silicones.

Des polymères à fonctions époxy sont commercialisés sous les dénominations CYRACURE® UVR-6110, CYRACURE® UVR-6105, CYRACURE® ERL-4221E, CYRACURE® ERL-4206, CYRACURE® UVR 6128, CYRACURE® UVR 6216 par la société UNION CARBIDE, DER® 439 par la société DOW CHEMICAL, les EPIKATES® 828,1001,1004,1007 de la société SHELL, l'ARALDITE® ECN1299 de la société CIBA-GEIGY, les EPOXYNOVOLACS® de la société DOW CHEMICAL.

### Les composés à doubles liaisons éthyléniques :

Les composés portant des doubles liaisons éthyléniques peuvent être de toute nature chimique. Ils peuvent notamment être choisis parmi :
a) les polyesters à insaturation(s) éthylénique(s) :
   Il s'agit d'un groupe de polymères de type polyester présentant une ou plusieurs doubles liaisons éthyléniques, réparties de manière aléatoire dans la chaîne principale du polymère. Ces polyesters insaturés sont obtenus par polycondensation d'un mélange
   - de diacides carboxyliques aliphatiques linéaires ou ramifiés ou cycloaliphatiques comportant notamment de 3 à 50 atomes de carbone, de préférence de 3 à 20 atomes de carbone, tels que l'acide adipique ou l'acide sébacique, de diacides carboxyliques aromatiques ayant notamment de 8 à 50 atomes de carbone, de préférence de 8 à 20 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
   - de diols aliphatiques linéaires ou ramifiés ou cycloaliphatiques comportant notamment de 2 à 50 atomes de carbone, de préférence de 2 à 20 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 atomes de carbone tel que le bisphénol A, le bisphénol B, et/ou de dimères diols issus de la réduction des dimères d'acides gras tels que définis précédemment, et
   - d'un ou de plusieurs diacides carboxyliques ou leurs anhydrides comportant au moins une double liaison éthylénique polymérisable et ayant de 3 à 50 atomes de carbone, de préférence de 3 à 20 atomes de carbone, tels que l'acide maléique, l'acide fumarique ou l'acide itaconique.
b) les polyesters à groupes (meth)acrylate latéraux et/ou terminaux :
   Il s'agit d'un groupe de polymères de type polyester obtenus par polycondensation d'un mélange
   - de diacides carboxyliques aliphatiques linéaires ou ramifiés ou cycloaliphatiques comportant notamment de 3 à 50 atomes de carbone, de préférence de 3 à 20 atomes de carbone, tels que l'acide adipique ou l'acide sébacique, de diacides carboxyliques aromatiques ayant notamment de 8 à 50 atomes de carbone, de préférence de 8 à 20 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
   - de diols aliphatiques linéaires ou ramifiés ou cycloaliphatiques comportant notamment de 2 à 50 atomes de carbone, de préférence de 2 à 20 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 atomes de carbone tel que le bisphénol A, le bisphénol B, et
   - d'au moins un monoester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
      Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.

   De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).
c) les polyuréthannes et/ou polyurées à groupes (méth)acrylate, obtenus par polycondensation
   - de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule 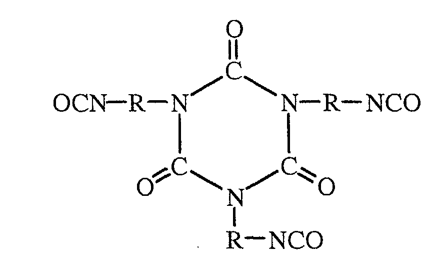 résultant de la trimérisation de 3 molécules de diisocyanates OCN-R-CNO,
   où R est un radical hydrocarboné linéaire, ramifié ou cyclique comportant de 2 à 30 atomes de carbone ;
   - de polyols, notamment de diols, exempts d'insaturations éthyléniques polymérisables, tels que le 1,4-butanediol, l'éthylèneglyco ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
   - d'au moins un monoester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

   De tels polyuréthannes/polyurées à groupes acrylates sont commercialisés par exemple sous la dénomination SR 368 (tris(2-hydroxyéthyl)isocyanuratetriacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
   On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.
d) les polyéthers à groupes (méth)acrylate obtenus par estérification, par l'acide (méth)acrylique, des groupes hydroxyle terminaux d'homopolymères ou de copolymères d'alkylèneglycols en C₁₋₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.
   Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre
   - au moins un diépoxyde choisi par exemple parmi :
      1) l'éther diglycidylique de bisphénol A,
      2 )une résine diépoxy résultant de la réaction entre l'éther diglycidylique de bisphénol A et l'épichlorhydrine,
      3) une résine époxyester à extrémités α,ω-diépoxy résultant de la condensation d'un diacide carboxylique ayant de 3 à 50 atomes de carbone avec un excès stoechiométrique de 1) et/ou 2), et
      4) une résine époxyéther à extrémités α,ω-diépoxy résultant de la condensation d'un diol ayant de 3 à 50 atomes de carbone avec un excès stoechiométrique de 1) et/ou 2),
      5) les huiles naturelles ou synthétiques portant au moins 2 groupes époxyde, telles que l'huile de soja époxydée, l'huile de lin époxydée et l'huile de vernonia époxydée,
      6) un polycondensat phénol-formaldéhyde (résine Novolac®), dont les extrémités et/ou les groupes latéraux ont été époxydés,
      et
   - un ou plusieurs acides carboxyliques ou polyacides carboxyliques comportant au moins une double liaison éthylénique en α,β du groupe carboxylique comme l'acide (méth)acrylique, l'acide crotonique ou les monoesters d'acide (méth)acrylique et d'un diol ou polyol ayant de 2 à 20 atomes de carbone, de préférence de 2 à 6 atomes de carbone tels que le (méth)acrylate de 2-hydroxyéthyle.

   De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.
f) les poly(méth)acrylates d'(alkyle en C₁₋₅₀) comportant au moins deux fonctions à double liaison éthylénique portées par les chaînes hydrocarbonées latérales et/ou terminales.
   De tels copolymères sont commercialisés par exemple sous les dénominations IRR® 375, OTA® 480 et EBECRYL® 2047 par la société UCB.
g) les polyorganosiloxanes à groupes (méth)acrylate ou (méth)acrylamide obtenus respectivement
   - par estérification, p. ex. par l'acide (méth)acrylique, de polyorganosiloxanes, de préférence de polydiméthylsiloxanes (PDMS), portant des groupes hydroxyle terminaux et/ou latéraux,
   - par amidification, par exemple par l'acide (méth)acrylique, de polyorganosiloxanes porteurs de groupes amine primaire ou secondaire latéraux et/ou terminaux.
      Comme PDMS hydroxylés on peut citer en particulier des PDMS comportant au moins deux groupes hydroxyalkyle en C₁₋₆ et les diméthicone-copolyols avec des groupes hydroxyle latéraux ou terminaux.
      Des polydiméthylsiloxanes α,ω-dihydroxylés estérifiables sont commercialisés sous les dénominations TEGOMER® H-Si 2111 et TEGOMER® H-Si 2311 par la société GOLDSCHMIDT. Des polydiméthylsiloxanes α,ω-diacrylate sont disponibles à la société SHIN-ETSU sous les références X-22-164 B et X-22-164C.
      Comme PDMS aminés, on peut citer en particulier des PDMS comportant au moins 2 groupes aminoalkyle en C₁₋₁₀, par exemple la silicone aminée commercialisée sous la dénomination Q2-8220 par la société DOW CORNING.
      Avantageusement, les polymères siliconés de ce groupe sont utilisés en mélange avec un ou plusieurs polymères des autres groupes a) à f) décrits ci-dessus, notamment pour modifier le caractère hydrophobe de la composition finale.
h) les perfluoropolyéthers à groupes acrylate obtenus par estérification, par exemple par l'acide (méth)acrylique, de perfluoropolyéthers portant des groupes hydroxyle latéraux et/ou terminaux.
   De tels perfluoropolyéthers α,ω-diols sont décrits notamment dans EP-A-1057849 et sont commercialisés par la société AUSIMONT sous la dénomination FOMBLIN® Z DIOL.
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.
   Les dendrimères (du grec *dendron =* arbre) sont des molécules polymères "arborescentes", c'est-à-dire très ramifiées inventées par D. A. Tomalia et son équipe au début des années 90 (Donald A. Tomalia *et al*., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.
   Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).
   La société PERSTORP commercialise sous la dénomination BOLTORN® des polyesters hyperramifiés. On trouvera sous la dénomination COMBURST® de la société DENDRITECH des polyéthylèneamines hyperramifiées. Des poly(esteramide) hyperramifiés à extrémités hydroxyle sont commercialisés par la société DSM sous la dénomination HYBRANE®.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

Dans un mode de réalisation préféré de l'invention, on utilisera par conséquent ces polymères dendritiques et hyperramifiés en association avec un ou plusieurs des polymères et/ou oligomères a) à h) ci-dessus.

Les fonctions réactives bloquées Y sont choisies notamment parmi les fonctions amines bloquées sous forme cétimine et aldimine, et les fonctions aminoalcool bloquées sous forme oxazolidine.
Les composés portant les fonctions amines bloquées sont notamment choisis :
a) parmi les polyamines bloquées de formule générale 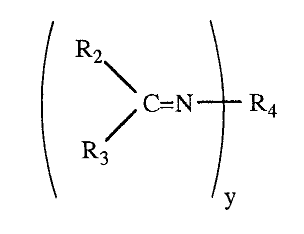 qui sont des imines résultant de la réaction entre un composé à groupes amines de formule R₄-(NH₂)_{y} et d'une cétone de formule 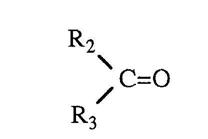 y étant supérieur ou égal à deux, de préférence y va de 2 à 100, R₂ égal ou différent de R₃ étant un groupe alkyle ayant de un à quatre atomes de carbone. De préférence R₂ = R₃ = -CH₃, ou R₂ = -CH₃ et R₃ = -C₂H₅, ou R₂ = -CH₃ et R₃ = isobutyle ou isopropyle.
   Le composé R₄-(NH₂)_{y} peut être une diamine, une polyamine, un oligomère, ou un polymère à groupes amines, choisis de préférence parmi
   1) les diamines aliphatiques, cycloaliphatiques ou aromatiques ayant notamment de 2 à 60 atomes de carbone, tel que l'éthylène diamine, le 1,2 diaminopropane, le 1,3 diaminopropane, le 1,4 diaminobutane, le 1,2 diamino-2-méthylpropane, le 1,6 diaminohexane, le 1,10 diaminodécane, l'isophorone diamine, l'adamantane diamine, la 2,6 diaminopyridine, ou les diamines obtenues par modification des extrémités d'acides gras dimères,
   2) les amines multifonctionnelles ayant plus de deux groupes amines telles que la mélanine, la 2,4,6 triaminopyrimidine, la 3,3' diaminobenzidine ou la 2,4,5,6 tétraaminopyrimidine,
   3) les oligomères porteurs d'au moins deux groupes amines tels que les diamines de polyalkylèneoxydes JEFFAMINE® de TEXACO (polyétherdiamines),
   4) les oligomères ou polymères (homopolymères et copolymères) porteurs de groupes amines de préférence primaires situés aux extrémités et/ou latéralement aux chaînes, en particulier les homo et copolymères de vinylamine ou allylamine, les polycondensats de toutes nature porteurs de groupes amines et en particulier les polyamides obtenus par condensation d'un excès de diamine,
   5) les dendrimères ou polymères hyperbranchés dont les extrémités de chaînes sont des amines primaires, en particulier les polyamidoamines tels que ceux vendus sous la dénomination STARBUST® par la société DENDRITECH. Les polymères hyperramifiés sont des polycondensats généralement de type polyéthylèneamine obtenus à partir de monomères multifonctionnels, qui ont une structure arborescente similaire à celle des dendrimères mais beaucoup moins régulière que celle-ci.

   Des composés à fonctions amines bloquées sous forme cétimine sont notamment vendus sous la dénomination Epikure® H3, Epikure® 3505 par la société SHELL, VESTAMIN® A139 par la société CREANOVA.
b) parmi d'autres composés portant des fonctions amines bloquées, notamment choisis parmi les polyamines bloquées de formule générale 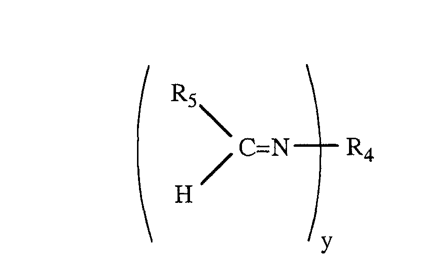 qui sont des imines résultant de la réaction entre un composé à groupes amines de formule R₄-(NH₂)_{y} et un aldéhyde de formule 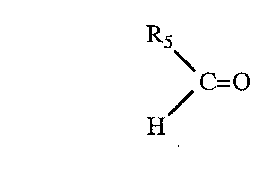 y étant un entier supérieur ou égal à deux, R₅ étant un groupe alkyle ayant de un à quatre atomes de carbone, R₄ ayant la même signification que précédemment.
c) parmi d'autres composés, portant des fonctions aminoalcools bloquées sous forme oxazolidine et notamment choisis parmi les composés de formule générale 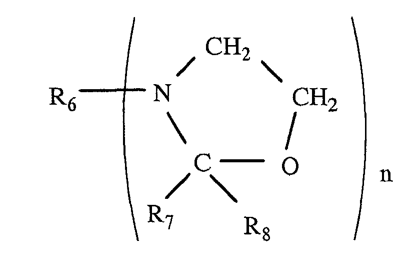 où R₆ est un groupe hydrocarboné comportant de un à cinquante atomes de carbones, linéaire, ramifié, cyclique ou hétérocyclique, pouvant comporter une ou plusieurs insaturations, et pouvant comporter un ou plusieurs hétéroatomes tel que O (pouvant notamment être sous forme de groupes polyalkylèneoxydes), S, N, P, Si, le groupe R₆ pouvant comporter d'autres fonctions réactives que les fonctions amines, ne réagissant pas avec les fonctions réactives non bloquées, R₆ pouvant également être un polymère ou un dendrimère ou un polymère hyperbranché,
   R₇ et R₈ étant égaux ou différents sont l'atome d'hydrogène ou un groupe alkyle comportant de un à quatre atomes de carbones, avec de préférence R₇= R₈=CH₃ ou R₇=CH₃ et R₈=C₂H₅, isopropyle ou isobutyle,
   n étant un entier positif supérieur ou égal à deux.

Ces composés sous forme oxazolidine, en particulier ceux pour lesquels n=2, sont notamment obtenus par condensation entre un composé comportant une ou plusieurs fonctions aminoalcool de formule R₆-(NH-CH₂-CH₂-OH)ₙ et une cétone de formule 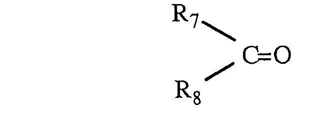 ou un aldéhyde de formule

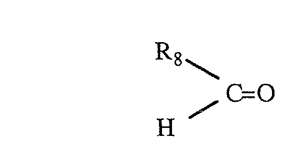

Ces composés peuvent également être obtenus en partant d'un diaminoalcool, qui est alors généralement la diéthanolamine, et en cyclisant en deux temps :
- cyclisation d'un groupe aminoalcool en présence d'une cétone (ou d'un aldéhyde), le deuxième groupe alcool n'étant pas touché par la réaction 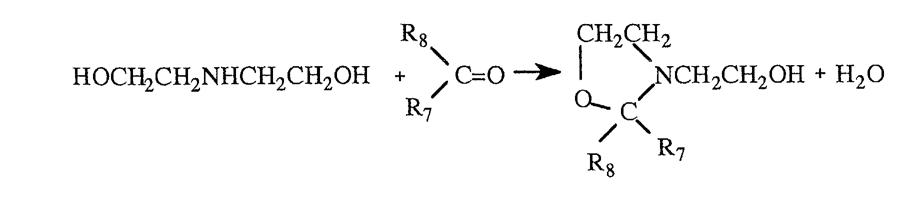
- puis réaction avec un coupleur possédant au moins deux groupes Y₁ pouvant réagir avec l'hydroxyle dans des conditions anhydres, ne permettant pas l'ouverture du cycle oxazolidine formé selon la réaction 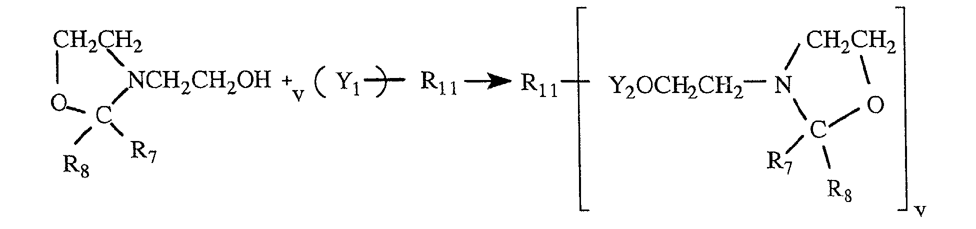 R₁₁ étant équivalent à R₄ défini précédemment,
   Y₂ étant le groupe formé par la réaction entre Y₁ et -OH
   Dans la réaction ci-dessus, le composé de formule 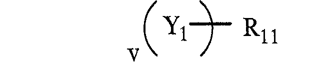 peut être :
   - un diisocyanate aliphatique, cycloaliphatique ou aromatique,
   - un trimère de diisocyanate,
   - un triisocyanate de formule (II),
   - un polyisocyanate résultant de la réaction entre un excès de diisocyanate et un polyol.

Dans chacun de ces cas, Y₁ = -NCO, et Y₂ est représenté par la formule

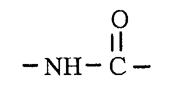

De la même façon, le composé de formule 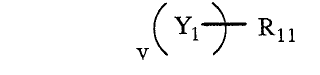 peut être un diacide, triacide ou polyacide ou ester ou chlorure d'acide, la réaction se faisant dans des conditions anhydres. Dans ce cas, Y₁ = -COOH ou -COC1, et Y₂ est représenté par la formule

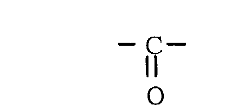

De la même facon, le composé de formule 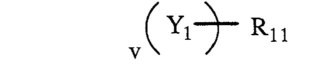 peut être un diépoxyde, triépoxyde ou polyépoxyde, la réaction se faisant dans des conditions anhydres. Dans ce cas, Y₁ est représenté par la formule 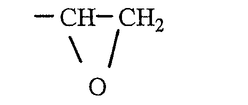 et

Y₂ = -CHOH-CH₂-

Selon un mode particulier de réalisation de l'invention, la composition peut comprendre au moins un composé comportant à la fois des fonctions réactives non bloquées X et des fonctions réactives bloquées Y. On citera à titre d'exemple les cétimines et aldimines ne bloquant que deux groupes hydroxyles d'un polyol comme le glycérol ou le triméthylolpropane, le ou les groupes hydroxyles restant réagissant ensuite avec un diisocyanate en excès, donnant ainsi un composé porteur à la fois de groupes cétimine (ou aldimine) et de plusieurs groupes isocyanates libres suivant : 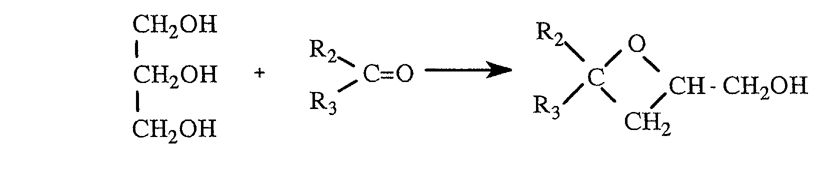 puis

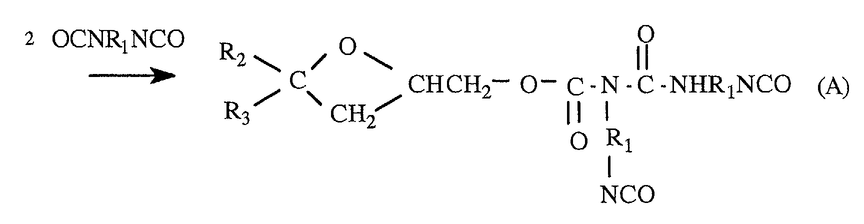

Le composé A formé possède à la fois un groupe cétimine ou aldimine débloquable à l'humidité, et un groupe réactif allophanate (c'est-à-dire porteur de deux fonctions réactives isocyanate).
Dans ce cas particulier, le composé A pourra, à l'application sur un support kératinique et en présence d'humidité, débloquer le groupe cétimine ou aldimine et co-réagir sur lui-même en donnant une réaction simultanée de polymérisation et de réticulation suivant :

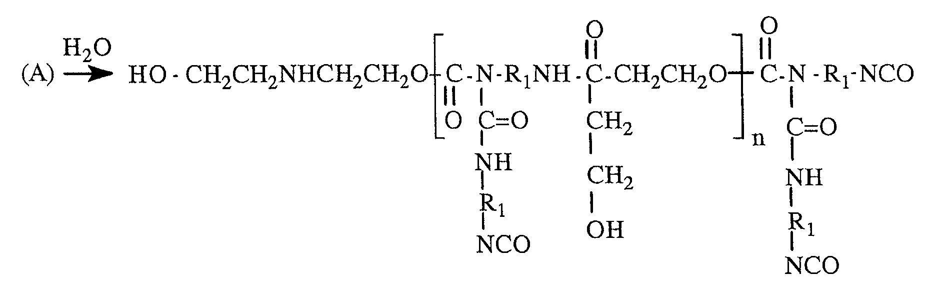

Les fonction -NCO et -OH assurent la co-réticulation avec une autre chaîne polymère formée et/ou sur le composé lui même, pour donner un réseau réticulé. Ces composés auto-réactifs A et leurs réactions sont notamment cités par H. RENZ, XXVI^{th} International Conference in Organic Coatings, Athenes, juillet 2000, p. 237-249, 2000.

Des composés à fonctions aminoalcools bloquées sous forme oxazolidine sont notamment décrits dans les documents WO-A-99/07763, JP-A-09-241501, WO-A-96/20231, WO-A-95/14528, US-A-5126421, US-A-4381388, US-A-4504647. Ils sont vendus sous les dénominations INCOZOL® 4, INCOZOL® LV par la société INDUSTRIAL COPOLYMER LTD, HARDENER® OZ par la société BAYER, ZOLDINE® RD-4 par la société ANGUS CHEMICALS CO.

Les fonctions amine ou hydroxyle correspondant aux fonctions cétimine, aldimine ou oxazolidine sont débloquées à l'humidité selon les réactions suivantes :

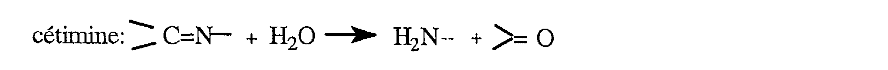

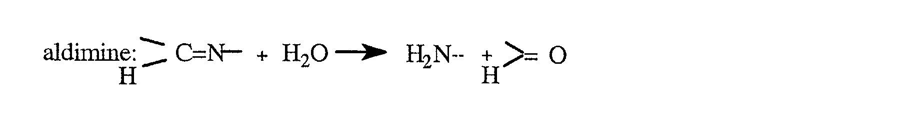

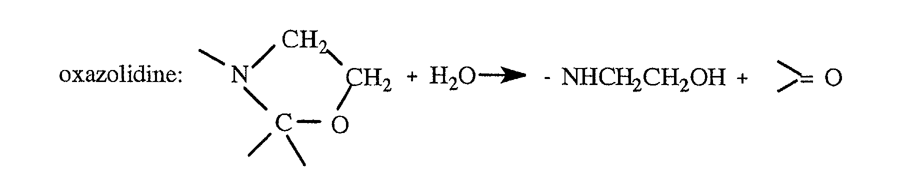

Les fonctions une fois débloquées réagissent de manière habituelle avec les fonctions N=C=O.
Les compositions cosmétiques réticulant à l'humidité de la présente invention contiennent donc de préférence un ou plusieurs catalyseurs capables d'accélérer la réaction de déblocage à l'humidité des fonctions isocyanate bloquées. Ces catalyseurs sont choisis notamment parmi les amines tertiaires, linéaires, ramifiées ou cycliques telles que le diazobicyclo[2,2,2]octane, la quinuclidine, le 3,3,6,9,9-pentaméthyl-2,10-diazabicyclo[4,4,0]dèc-1-ène.

Selon la présente invention, la concentration des catalyseurs est de préférence comprise entre 0,1 et 2 % en poids par rapport aux réactifs, et de préférence entre 0,2 et 1 % en poids.

Les composé A et B, ou le(s) composé(s) comportant à la fois au moins deux fonctions réactives non bloquées et au moins deux fonctions réactives bloquées, peuvent être présents dans la composition selon l'invention en une teneur allant de 1 à 50% en poids, par rapport au poids total de la composition, et de préférence allant de 2 à 40% en poids. De préférence, le rapport du nombre total de fonctions réactives bloquées Y sur le nombre total de fonctions réactives non bloquées X est supérieur à 1, et notamment va jusqu'à 1,5.

Les compositions cosmétiques réticulant à l'humidité de la présente invention peuvent contenir en outre un ou plusieurs solvants organiques. Ces solvants sont notamment choisis parmi les solvants organiques physiologiquement acceptables parmi lesquels on peut citer
b ) les cétones liquides à température ambiante telles que la méthyléthylcétone, la méthylisobutylcétone, la diisobutylcétone, l'isophorone, la cyclohexanone et l'acétone,
c) les alcools liquides à température ambiante tels que l'éthanol, l'isopropanol, le diacétone-alcool, le 2-butoxyéthanol ou le cyclohexanol,
d ) les glycols liquides à température ambiante tels que l'éthylèneglycol, le propylèneglycol, le pentylèneglycol et le glycérol,
e) les éthers de propylèneglycol liquides à température ambiante tels que le monométhyléther de propylèneglycol, l'acétate de l'éther monométhylique de propylèneglycol, le mono-n-butyléther de dipropylèneglycol,
f) les esters à courte chaîne (comportant au total de 3 à 8 atomes de carbone) tels que l'acétate d'éthyle, l'acétate de méthyle, l'acétate de propyle, l'acétate de n-butyle et l'acétate d'isopentyle,
g) les alcanes liquides à température ambiante tels que le décane, l'heptane, le dodécane, le cyclohexane,
h) les hydrocarbures aromatiques liquides à température ambiante tels que le toluène et le xylène,
i) les silicones liquides à température ambiante et
j) des mélanges de ceux-ci.

La teneur en solvant dans la composition peut aller de 0 % à 80 % en poids, par rapport au poids total de la composition, et de préférence de 1 à 60 % en poids.
Selon un mode particulier de la composition selon l'invention, la composition est exempte de solvant.

Les réactifs peuvent être dissous au départ dans tout type de solvant organique volatil, ne comportant pas d'atomes d'hydrogène labiles et exempt d'eau.

Ainsi, pour une meilleure conservation de la formule vis-à-vis de traces d'eau, on peut y incorporer selon un mode de réalisation de l'invention un absorbeur d'humidité, qui peut être de toute nature chimique, de préférence solide sous forme de microparticules comme la silice, le talc, la kaolin, les silicates d'alumine, les terres de diatomées.

Les additifs sont choisis de telle sorte qu'ils ne réagissent pas avec les fonctions réactives non bloquées X et les fonctions réactives bloquées Y des composés réticulant à l'humidité.

Les compositions précités peuvent être utilisées comme vernis à ongle.

L'invention a également pour objet un procédé de revêtement des matières kératiniques. Ce procédé comprend l'application sur un substrat kératinique d'une couche d'une composition cosmétique réticulant à l'humidité décrite ci-dessus. Sous l'action de l'humidité provenant de l'air et/ou du support, les fonctions réactives bloquées Y se débloquent et réagissent avec les fonctions non bloquées X, permettant l'obtention d'un film réticulé partiellement ou totalement sur le substrat.

Les substrats kératiniques susceptibles de recevoir des revêtements thermoréticulables selon la présente invention sont en particulier les ongles, les cils, les sourcils et les cheveux, ou les accessoires de maquillages tels que les faux-ongles, le faux-cils ou les postiches.

L'invention est décrite plus en détail par les exemples ci-dessous.

### Exemples

### Exemple 1 :

On a préparé un vernis à ongles réticulable à l'humidité de l'air ayant la composition suivante :
- polymère à fonction époxyde (Epikote® 828 de Shell) 21,6 g
- composé à fonctions amine bloquées sous forme cétimine
   (Epikure® H3 de Shell) 8,4 g
- silice pyrogénée (Aerosil® 200 de Degussa) 2,0 g
- pigment deshydraté 3,0 g
- toluène déshydraté 40 g
- méthyl isobutyl cétone deshydraté qsp pour 100 g

On applique la composition sur les ongles sous forme d'un film qui se réticule au contact de l'humidité de l'air. Après séchage, le film sec formé présente de bonnes propriétés d'adhésion, de brillance et de tenue dans le temps.

### Exemple 2 :

On a préparé un vernis à ongles réticulable à l'humidité de l'air ayant la composition suivante :
- polymère à fonction époxyde (Epikote® 215 de Shell) 21 g
- composé à fonctions amine bloquées sous forme cétimine
   (Epikure® 3505 de Shell) 9 g
- silice (Syloïd® 72 de Grace) 2 g
- poudre de diamant 0,001 g
- céramide 0,001 g
- éthanol deshydraté 5 g
- heptane deshydraté 30 g
- methyl ethyl cétone deshydraté qsp pour 100 g

On applique la composition sur les ongles sous forme d'un film qui se réticule au contact de l'humidité de l'air. Après séchage, le film sec formé présente de bonnes propriétés d'adhésion, de brillance et de tenue dans le temps.

## Revendications

1. Utilisation comme vernis à ongle d'une composition cosmétique réticulant à l'humidité comprenant, dans un milieu anhydre cosmétiquement acceptable, et exempt de composés à atomes d'hydrogène labiles,
i) soit
a) au moins un premier composé, appelé composé A, comportant au moins deux fonctions réactives non bloquées X, et
b) au moins un deuxième composé, appelé composé B comportant au moins deux fonctions réactives bloquées Y, susceptibles d'être débloquées sous l'action de l'humidité de manière à pouvoir réagir avec les fonctions réactives non bloquées X du ou des composés A,
ii) soit au moins un composé comportant à la fois au moins deux fonctions réactives non bloquées X et au moins deux fonctions réactives bloquées Y, susceptibles d'être débloquées sous l'action de l'humidité de manière à pouvoir réagir avec les fonctions réactives non bloquées X,
la fonctionnalité moyenne du système, c'est-à-dire le nombre total de fonctions réactives non bloquées X et de fonctions réactives bloquées Y rapporté au nombre total de molécules de composés étant strictement supérieure à 2.

2. Utilisation selon la revendication 1, **caractérisée par le fait qu'**une partie ou la totalité des composés A portent également une ou plusieurs fonctions réactives bloquées Y et/ou qu'une partie ou la totalité des composés B portent également une ou plusieurs fonctions réactives non bloquées X.

3. Utilisation selon la revendication 1 ou 2, **caractérisée par le fait que** l'ensemble des composés A et B comporte à la fois des fonctions réactives non bloquées X et des fonctions réactives bloquées Y.

4. Utilisation selon l'une quelconque des revendications précédentes, **caractérisée par le fait que** l'humidité provient de l'air et/ou du support sur lequel est appliquée ladite composition.

5. Utilisation selon l'une quelconque des revendications précédentes, **caractérisé par le fait que** les fonctions réactives non bloquées X sont choisies parmi les fonctions isocyanate et époxyde, et les doubles liaisons éthyléniques.

6. Utilisation selon l'une quelconque des revendications précédentes, **caractérisée par le fait que** les fonctions réactives bloquées Y sont choisies parmi les fonctions amines bloquées sous forme cétimine et aldimine, et les fonction aminoalcool bloquées sous forme oxazolidine.

7. Utilisation selon l'une quelconque des revendications précédentes, **caractérisée par le fait qu'**elle comprend en outre un ou plusieurs solvants organiques.

8. Utilisation selon l'une quelconque des revendications précédentes, **caractérisée par le fait qu'**elle comprend en outre des catalyseurs en proportions comprises entre 0,1 et 2% en poids par rapport aux réactifs, de préférence entre 0,2 et 1% en poids.

9. Utilisation selon l'une quelconque des revendications précédentes, **caractérisée par le fait que** les composés A et B ou le(s) composé(s) portant à la fois au moins deux fonctions réactives non bloquées et deux fonctions réactives bloquées sont présents dans la composition en une teneur allant de 1 à 50% en poids par rapport au poids total de la composition, de préférence allant de 2 à 40% en poids.

10. Utilisation selon l'une quelconque des revendications précédentes, **caractérisée par le fait qu'**elle comprend en outre un absorbeur d'humidité.

11. Composition cosmétique réticulant à l'humidité comprenant, dans un milieu anhydre cosmétiquement acceptable, et exempt de composés à atomes d'hydrogène labiles,
i) soit
a) au moins un premier composé, appelé composé A, comportant au moins deux fonctions réactives non bloquées X, et
b) au moins un deuxième composé, appelé composé B comportant au moins deux fonctions réactives bloquées Y, susceptibles d'être débloquées sous l'action de l'humidité de manière à pouvoir réagir avec les fonctions réactives non bloquées X du ou des composés A, les fonctions réactives bloquées Y étant choisies parmi les fonctions amines bloquées sous forme aldimine et les fonctions aminoalcool bloquées sous forme oxazolidine,
ii) soit au moins un composé comportant à la fois au moins deux fonctions réactives non bloquées X et au moins deux fonctions réactives bloquées Y, susceptibles d'être débloquées sous l'action de l'humidité de manière à pouvoir réagir avec les fonctions réactives non bloquées X,
la fonctionnalité moyenne du système, c'est-à-dire le nombre total de fonctions réactives non bloquées X et de fonctions réactives bloquées Y rapporté au nombre total de molécules de composés étant strictement supérieure à 2.

12. Composition cosmétique réticulant à l'humidité selon la revendication 11, caracérisée par le fait qu'une partie ou la totalité des composés A portent également une ou plusieurs fonctions réactives bloquées Y et/ou qu'une partie ou la totalité des composés B portent également une ou plusieurs fonctions réactives non bloquées X.

13. Composition cosmétique réticulant à l'humidité selon la revendication 11 ou 12, **caractérisée par le fait que** l'ensemble des composés A et B comporte à la fois des fonctions réactives non bloquées X et des fonctions réactives bloquées Y.

14. Composition cosmétique réticulant à l'humidité selon l'une quelconque des revendications 11 à 13, **caractérisée par le fait que** l'humidité provient de l'air et/ou du support sur lequel est appliquée ladite composition.

15. Composition cosmétique réticulant à l'humidité selon l'une quelconque des revendications 11 à 14, **caractérisé par le fait que** les fonctions réactives non bloquées X sont choisies parmi les fonctions isocyanate et époxyde, et les doubles liaisons éthyléniques.

16. Composition cosmétique réticulant à l'humidité selon l'une quelconque des revendications 11 à 15, **caractérisée par le fait que** les fonctions réactives bloquées Y du composé comportant à la fois au moins deux fonctions réactives non bloquées X et au moins deux fonctions réactives bloquées Y sont choisies parmi les fonctions amines bloquées sous forme cétimine et aldimine, et les fonction aminoalcool bloquées sous forme oxazolidine.

17. Composition cosmétique réticulant à l'humidité selon l'une quelconque des revendications 11 à 16, **caractérisée par le fait qu'**elle comprend en outre un ou plusieurs solvants organiques.

18. Composition cosmétique réticulant à l'humidité selon l'une quelconque des revendications 11 à 17, **caractérisée par le fait qu'**elle comprend en outre des catalyseurs en proportions comprises entre 0,1 et 2% en poids par rapport aux réactifs, de préférence entre 0,2 et 1% en poids.

19. Composition cosmétique réticulant à l'humidité selon l'une quelconque des revendications 11 à 18, **caractérisée par le fait que** les composés A et B ou le(s) composé(s) portant à la fois au moins deux fonctions réactives non bloquées et deux fonctions réactives bloquées sont présents dans la composition en une teneur allant de 1 à 50% en poids par rapport au poids total de la composition, de préférence allant de 2 à 40% en poids.

20. Composition cosmétique réticulant à l'humidité selon l'une quelconque des revendications 11 à 19, **caractérisée par le fait qu'**elle comprend en outre un absorbeur d'humidité.

21. Procédé de revêtement d'un substrat kératinique, **caractérisé en ce que** l'on applique sur ledit substrat une composition définie dans l'une quelconque des revendications 1 à 10.
